# EUROPEAN PATENT APPLICATION

(11) **EP 2 446 865 A1**
(43) Date of publication of application: **02.05.2012**
(21) Application number: 10189306.3
(22) Date of filing: 28.10.2010
(51) Int. Cl.: A61F 7/02, A61F 7/00

(54) **Thermostimulation apparatus**

(71) Applicant: Mohn, Louise, London W8 7RZ (GB)
(72) Inventor: Mohn, Louise, London W8 7RZ (GB)
(74) Representative: Jacob, Reuben Ellis

(57) **Abstract**

The present invention relates to a thermostimulation apparatus comprising means for providing heating and cooling and to pads for the application of electromagnetic stimulation, heating and/or cooling for use with the thermostimulation apparatus.

## Description

The present invention relates to a thermostimulation apparatus. More specifically, the present invention relates to a thermostimulation apparatus capable of providing both heating and cooling.

For a variety of therapeutic applications, several treatment modalities are currently known in the art including electrical stimulation, heat therapy and thermostimulation. Electrical stimulation involves the application of an electrical current to a single muscle or a group of muscles. The resulting contraction can produce a variety of effects from strengthening injured muscles and reducing edema to relieving pain and promoting healing.

Many electrical stimulation systems are limited to two to four channels and therefore allow only two to four pads to be applied to a patient. The pads are usually quite small and typically powered with a battery. This results in the application of a small amount of power and a low treatment depth of the resulting electric field. The shallow depth of the electric field generated by conventional electrical stimulation systems limits performance and patient benefit. Some systems have attempted to address this limitation by applying more current, often from a line or main supply source. However, the small size of conventional electrical stimulation pads is such that on the application of larger amounts of power, i.e. the use of higher currents, patients often report the experience of pain or discomfort.

Heat therapy itself is very useful as it has a number of effects such as relaxation of muscle spasm and increased blood flow that promotes healing. However, combination therapy, i.e. the synergistic use of other modalities such as massage, ultrasound and/or electrical stimulation has been found to be more effective than heat therapy alone.

Thermostimulation is one such combination therapy that involves the use of heat therapy and electrical stimulation simultaneously. With thermostimulation, the healing benefits of heat are provided along with the strengthening, toning, pain relieving and healing benefits of electrical stimulation. Moreover, the application of heat has been found effective in that it allows the patient to tolerate higher currents. This yields higher electric fields strengths, greater depths of penetration and therefore, more positive results than could be achieved with electrical stimulation without heat.

Therapeutic hypothermia can also be used for treatment of different conditions either as a therapeutic or protective measure. Local injuries or pain are often treated with heating, cooling or a combination of both to speed up the healing process. In practice, the administration of therapeutic hypothermia involves the application of cooling blankets or wraps with a water-circulation technology. These are susceptible to leakage and are not recommended for use in combination with electrically powered equipment.

Hence, a need remains in the art for an improved apparatus or method for thermostimulation therapy that is more safe and effective, and which can provide therapeutic treatment with the application of both heating and cooling.

According to a first aspect of the invention, there is provided a thermostimulation apparatus comprising means for providing heating and cooling.

In a preferred embodiment, the thermostimulation apparatus comprises at least one heating pad and at least one cooling pad.

Preferably, the at least one heating pad comprises means for providing electrical stimulation and means for providing heat. The heating pad may comprise a top layer, a bottom layer for contacting the area to be treated, and a first and a second middle layer for supporting the stimulation means and the heating means.

Preferably, the cooling pad comprises means to receive a refrigerant in a liquid state or in a gaseous state.

In another preferred embodiment, the thermostimulation apparatus comprises at least one pad capable of providing both heating and cooling.

The combined heating and cooling pad preferably comprises one or more Peltier elements. The pad may further comprise heat sink means and/or heat exchanger means.

Preferably, the pads according to the invention comprise a thermocouple connected to a central processing unit to regulate the temperature of the pad.

According to a second aspect of the invention, there is provided a heating pad, a cooling pad and a combined heating and cooling pad for use with a thermostimulation apparatus as described above.

The invention will be further described with reference to the drawings and figures, in which:
figure 1 is a block diagram of a thermostimulation apparatus according to the invention;
figure 2 is a flow chart showing the different parts of the thermostimulation apparatus according to the invention;
figure 3 is a diagram of a heating and electrical stimulation pad for use with the thermostimulation apparatus according to the invention;
figures 4A, 4B, 4C and 4D are schematic representation of the three layers of the heating and electrical stimulation pad of figure 3, as seen from the top;
figure 5A, 5B, 5C and 5D are schematic representations of the three layers of the heating and electrical stimulation pad of figure 3 as seen from the bottom;
figure 6 is a schematic representation of the layers of figures 5B and 4C;
figure 7 is a schematic representation of an apparatus according to the invention with a cooling pad;
figure 8 is a schematic representation of the top view of a middle layer of a cooling pad as shown in figure 7;
figure 9 is a schematic representation of the top view of a combined heating and cooling pad according to the invention; and
figure 10 is a schematic representation of the bottom view of a combined heating and cooling pad according to the invention.

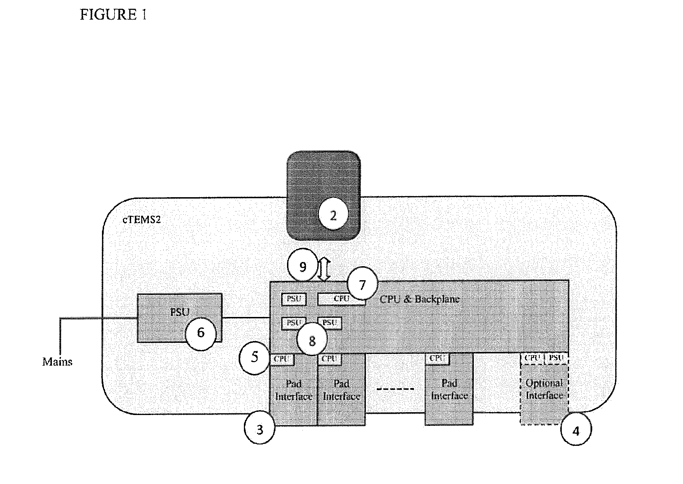

Referring to figure 1, there is illustrated a thermostimulation apparatus 1 (or "cTEMS2" apparatus) according to the invention. The apparatus may comprise a display and control unit 2 (for example an iPad®).

The apparatus 1 may also comprise one or more, and preferably up to twenty, interface cards 3 and optional interface cards 4. Each interface card 3 is adapted to receive a pad for the application of electromagnetic stimulation, heating and/or cooling to a patient. Preferably, each interface card 3 has its own Central Processing Unit (or "CPU") 5. Each interface card 3 may be adapted to receive bio-feedback from thermocouples or temperature sensors such as Pt1000® or Ptl00@ located the pad(s). The apparatus 1 may further comprise one or more optional interface cards 4 for example for bio-impedance measurements 16 for bio-feed back control of tissue properties for optimizing the effect output to the pad, for ultrasound 17, laser 18 and RF/microwave treatments.

The apparatus is preferably powered from the mains 6, and all the accessories connected to the interface cards 3, 4 may be powered from the mains 6 via the apparatus 1 through Power Supply Units (or "PSU") 8.

In use, a further Central Processing Unit (or "CPU") 7 receives protocol messages from the display and control unit 2 via Blue Tooth® or Wi-Fi® connection 9, interprets these messages and distributes the settings to the appropriate interface(s) or pad(s). The CPU 7 may also receive messages and alarm signals from the interface(s) or pad(s), act on these messages, and send status information to the user interface 2.

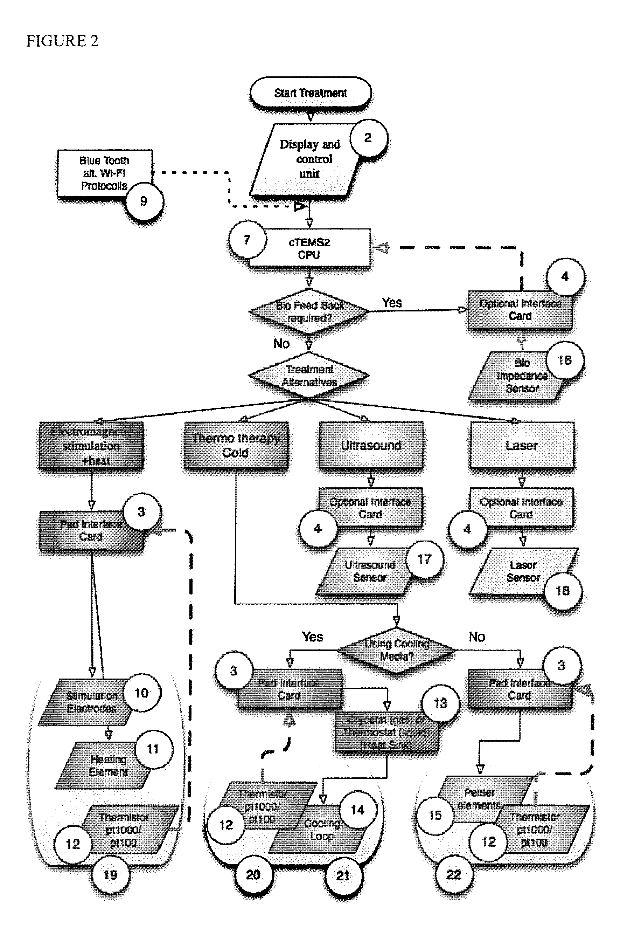

Figure 2 shows a flow chart providing an overview of an apparatus according to the invention and each part will be described in further detail below. Three types of pads are illustrated, namely a combined electromagnetic muscle stimulation and heating pad 19, a cooling pad using either cryogenic technology 20 or liquid in combination with a heat sink 21, a combined cooling and heating pad using Peltier elements 22.

The pad interface drives the currents and voltages needed to provide the wanted electromagnetic (EM) pulses at the pad. It also monitors the actual currents and voltages, and limits these at maximum values. In addition the pad interface delivers the current needed for heating. It is extremely important that the individual pad interfaces are electrically isolated. If not, the current may be driven between pads, resulting in a current through the body. The design includes a pre-set hardwired current limiting.

A microcontroller may be added to each pad and/or optional interface. This enables the use of a bus solution with a protocol for communication to the CPU. The need for cabling is thus greatly reduced. This microcontroller may also monitor EM voltage/current and limit these appropriately. An alarm is raised if limits are overridden. Normal operation for the pad interface for stimulation and heat 19 is preferably to continuously cycle the pulse sequence stored in RAM until otherwise instructed. The pulse sequences are downloaded to the interface via for example I²C. A set of commands, also via I²C, enables the activation of new pulse sequences, reading of temperature values and status, and so forth.

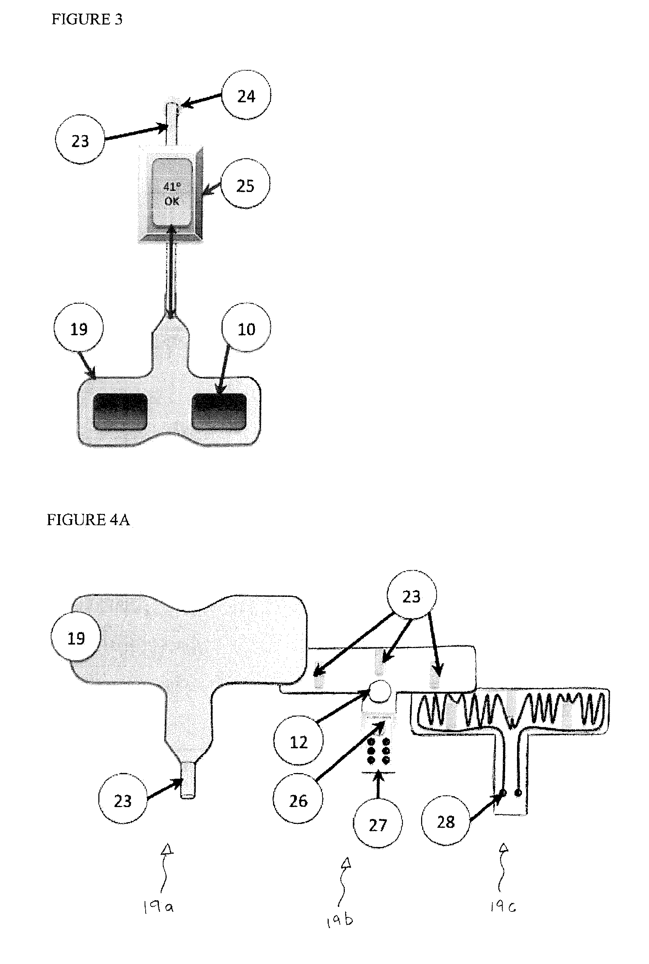

Figure 3 shows a pad 19 for use with the thermostimulation apparatus of the present invention adapted to provide heat and electromagnetic stimulation to a patient.

If the device has no functionality built in for feedback control of temperature via input from a thermocouple in the pad, a shielded pad cable 23 may be supplied with a with a connector 24, for example a 4-pin connector, for connection to the device and a temperature controlling unit 25 between the pad and the apparatus. The LCD display of this unit will indicate when the pad is connected, heating, the set point, and when the set point is reached as well as errors. Failure will be alarmed visually by error messages and with a buzzer. The system may be set up so that in case of either software or hardware failure current to the heating pads will be cut.

Preferably the pad comprises a temperature feedback control means, for example an external temperature controller device is added between the apparatus and the pad. If the apparatus has a built in temperature control means, then there is usually no need for the external temperature controller device 25 and the cable 23 may be mounted for example with a 6-pin connector 24 as further explained below. An 8-pin connector may be used to reduce assembly costs.

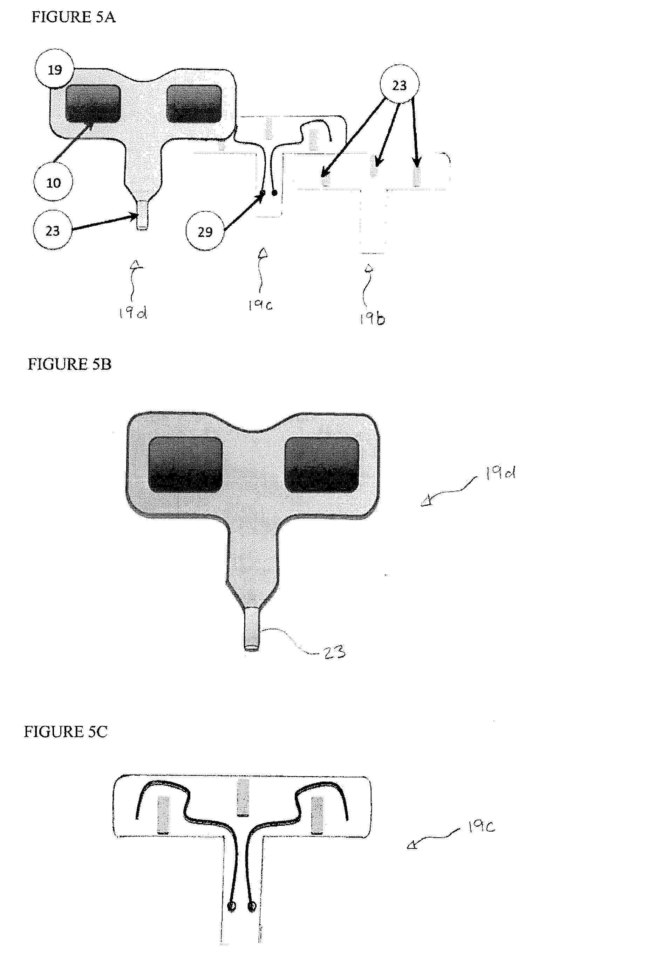

With reference to figures 4A-4D and figures 5A-5D, the heating and stimulation pad 19 may comprise four layers, namely a top layer 19a, a first middle layer 19c, a second middle layer 19d and a bottom layer 19d.

Within the context of this application, the expression "bottom layer" refers to the layer which is in contact with the skin of the patient; the expression "top layer" refers to the outermost layer (i.e. the furthest away from the skin of the patient) and the expression "middle layer" refers to any layer located between the bottom and the top layer. The expression "top surface" refers to the surface, in use, facing away from the patient; and the "bottom surface" to the surface, in use, facing towards from the patient

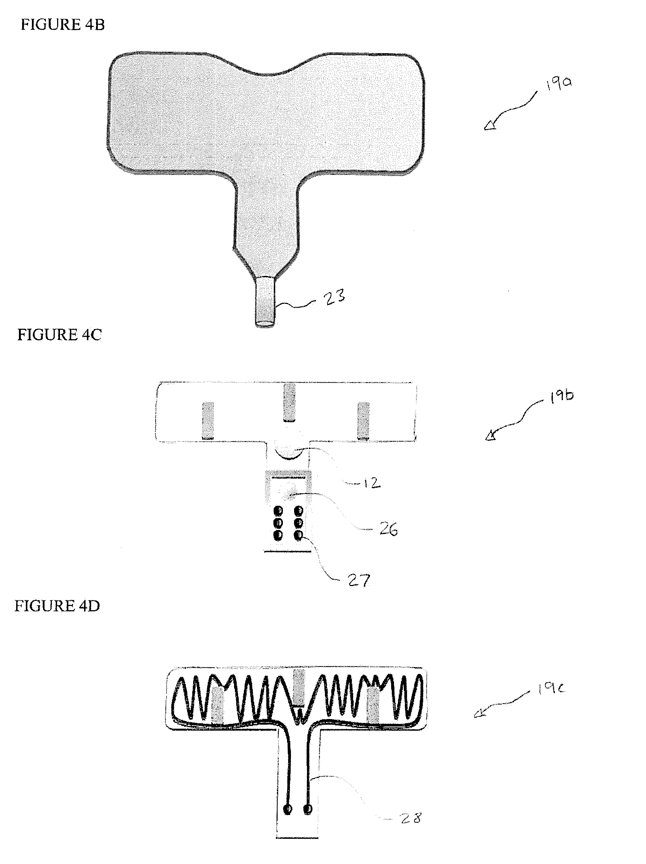

The top layer 19a (see for example figure 4) preferably comprises a nonconductive thermoplastic polymer so that the electromagnetic stimulation and heat are directed towards the patient and there are no losses.

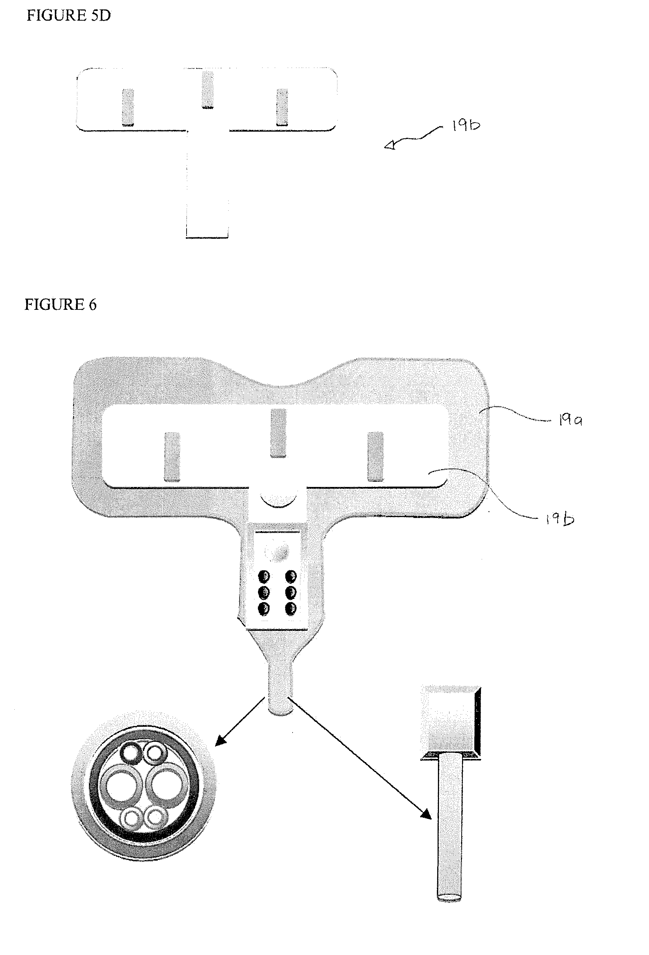

The first middle layer 19b (see figure 4C for top surface and figure 5D for bottom surface) preferably comprises a silicon polymer, a thermoplastic layer or one or more flexible print (PCBs). Thermoplastic polymers are preferred to silicon polymers because they are easier to manipulate during the moulding process. In addition, silicon polymers are a tendency to not stick as well as thermoplastic polymers, which is why it is preferred to include one or more holes in the middle layers to improve adhesion of the top and bottom layers. The top surface may comprise a thermocouple 12 (for example Pt1000®) which may be covered with silicon rubber; a LED 26, which may be used to indicate proper connection to the thermostimulation apparatus; and a connector 27, for example a 6 points connector, for mounting the cable 23 to the thermocouple 12, to the stimulation electrodes 10 and to the heating element 28.

The second middle layer 19c (see figure 4D for top surface and figure 5C for bottom surface) preferably comprises a silicon polymer. The top side of the second middle layer 19c may comprise the heating element 28, which is preferably a twisted wire glued to the surface. One or more stimulation wires 29 may be glued to the bottom surface of the second middle layer 19c.

The bottom layer 19d (see figure 5B) may comprise a nonconductive thermoplastic polymer. The bottom side of the bottom 19c may comprise one or more (preferably two) stimulation electrodes that preferably comprise conductive thermoplastic polymer.

The pads described in this application are preferably produced by injection moulding of pre-designed plastic polymers. Preferably, thermoplastic polymers are used for maximum control of the moulding process, quality (surface etc.), and bonding properties. Both middle layers preferably comprise a plurality, preferably three, elongated holes 23 to secure proper bonding between the top and the bottom layers 19a, 19d in the moulding process.

Figure 6 is a schematic top view of a bottom layer 19c with a second middle layer (not seen) and a first middle layer 19b. A print or soft print (PCB) may be provided on the top surface of the first middle layer 19b with a LED 26, which in use is visible through the top layer 19a and indicating when the pad 19 is properly connected to the thermostimulation apparatus.

Preferably, the print comprises a 6 points connector 27, i.e. two for the heating element 28, two for the stimulation electrodes 10 and two for the thermocouple 12. Figure 6 includes a cross-sectional view of the cable 23 (see bottom left), with two wires for the heating element 28, two for the stimulation electrodes 10 and two for the thermocouple 12. Figure 6 also includes a schematic view (bottom right) of a cable 23 mounted with a connector for stabilization during the moulding process. Polyurethane (PU) cables 23 are preferred for maximum bonding to pads to prevent cracks that allow water to enter the pads and create short cuts.

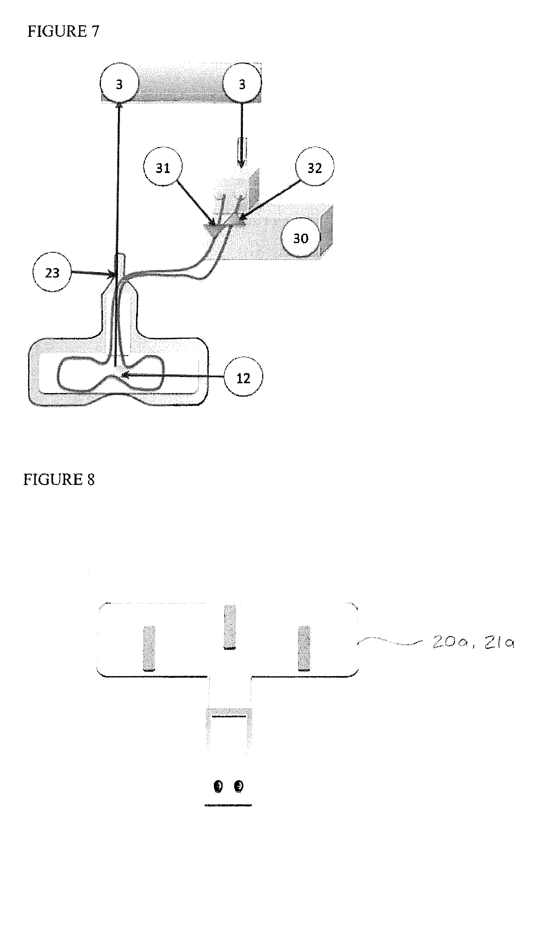

Figure 7 is a diagram illustrating the principle of the cooling pads 20, 21 according to the invention. Each cooling pad 20, 21 is adapted to receive using a cooling media or refrigerant and the control unit may regulate the supply of refrigerant to each cooling pad. The cooling device is preferably external to the thermostimulation apparatus and involves either gas (for example a cryogenic cooler), or a liquid cooler (for example a cooling bath with thermostat control). For both systems, the cooling pads may be flushed by a cooling media (gas or liquid) from a cooling device 30 through a feeding conduit 31 and a return conduit 32.

For the cryogenic system 20, the refrigerant is a liquid forced to vaporize in the cooling pad 20, it will then condensate in a heating sink within the cryogenic cooler 30. The refrigerant may be water, or preferably water with an additive such as iso-propanol to prevent freezing in the cooling system. The refrigerant is forced to vaporize in a reservoir in the cooling device, and cryogenic vapor is feed to the cooling pad through the feeding conduit 31. Alternatively, the refrigerant may be feed through the feeding conduit 31 to the cooling pad, and forced to vaporize in the cooling pad. Either way, the resultant vapor is returned to the apparatus via a return conduit 32. Alternatively, the liquid refrigerant is Argon or liquid Nitrogen.

When using the liquid system 21, there is no change in the physical state in the loop. Both external cooling systems may be controlled by feedback from thermocouple 12 through the cable 23 to the pad interface 3.

The cooling pad according to the invention preferably comprises a top layer, a middle layer and a bottom layer. Both the top and bottom layers comprising plastic polymer, preferably "cooling plastic polymers" and/or high heat conductive thermoplastic polymers for uniform distribution of the temperature.

The middle layer 20a, 21 a comprising a plastic polymer, preferably a thermoplastic low conductivity polymer and has a top surface and a bottom surface. Preferably one surface (see figure 8) of the middle layer 20a, 21a comprises an electrical connection to the thermocouple 12 via cable 23 and the other surface (see figure 7) comprises one or more glued conduits 31, 32 (preferably in polyurethane) in fluid connection with the external cooling system.

A thermocouple 12 is preferably placed on the cooling surface where the conduit 31, 32 is located. The cable 23 is preferably covered with polyurethane, shielded and only two wires are needed. Feedback is sent from the thermocouple 12 to the thermostimulation apparatus and the temperature may be regulated so that the skin temperature does not decrease below 1°C. A control unit connected to the thermocouple 12 may be used to regulate the refrigerant flow and thus the temperature of the cooling pad. Preferably the surface temperature of the cooling pad is higher than 1 °C, and preferably less then 42°C. If required, a heating means may be provided in the thermostimulation apparatus or in the cooling pad, for further regulation of the refrigerant temperature in the cooling pad.

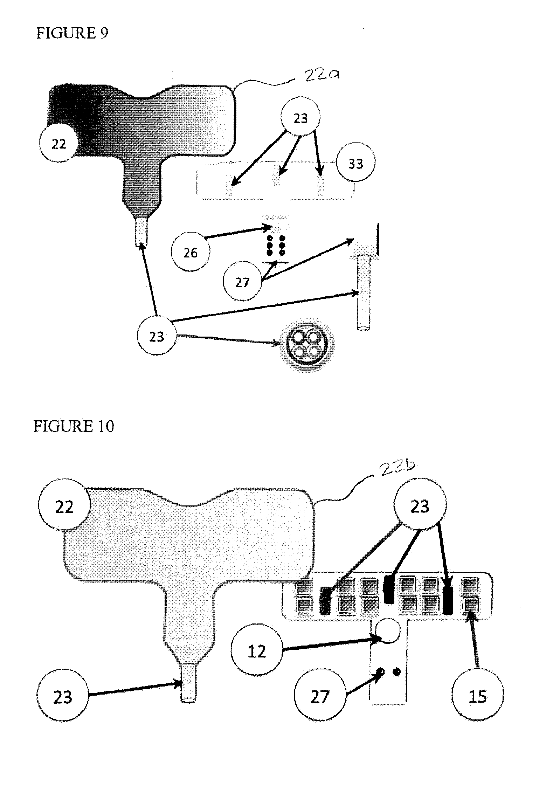

Figure 9 is a diagram illustrating the principle of the combined heating and cooling pads 22 according to the invention, using one or more Peltier effect thermoelectric elements. In this embodiment, there is no need for an external cooling device, as the temperature can be controlled by feedback from a thermocouple 12 similar to that described above in relation to the heating and stimulation pads. By switching polarity, the pads 22 can be used for heating. The temperature difference between the two sites is typically up to 60°C. Therefore, the pad was designed to allow for maximum heath dissipation from the surface of the top layer 22a as outlined in Figures 9 and 10. Heating and cooling temperature may be pre-selected and controlled using suitable control means.

The combined heating and cooling pad preferably comprises a top layer 22a, a middle layer 33 and a bottom layer 22b. Figure 9 is a schematic top view of the top layer 22a and the middle layer 33 and figure 10 is a schematic bottom view of the bottom layer 22b and the middle layer 33.

Preferably, the top layer 22a comprises a heat sink means, and/or "cooling plastic polymers" or high heat conductive thermoplastic polymers for maximum dissipation of heat. Alternatively, the heat sink means comprises an air cooled fin array (for example a larger surface on the top side with a heat conductive polymer) or liquid in adjacent the top layer 22a. The liquid may for example be locatedin the pocket or jacket and connected to a cooling system. Since the difference between the top and bottom surfaces can be approximately 60 degrees, it implies that the top surface temperature would be more than 60 degrees and need to be dissipated.

The combined heating and cooling pad may comprise a heat exchanger means to establish heat exchange between the Peltier element and the patient's skin. The heat exchanger means may comprise comprises a heat fluid flow path and/or a thermal contact pad for establishing thermal contact with the patient's skin.

The middle layer 33 may comprise one or more holes 23 to obtain maximum bonding between the top and bottom layers 22a, 22b in the moulding process. The top and bottom layers 22a, 22b preferably comprise non- or low conductive thermoplastic polymer.

The bottom surface of the middle layer 33 may comprise one or more Peltier elements 15 and may further comprise a thermocouple covered with plastic polymers 12.

The top surface of the middle layer 33 may comprise a connector 27 and the LED 26. Four welding/connector points (i.e. the connector 27) for the Peltier elements are shown, but not the wires connecting the elements. The connector 27 may be attached to the welding/connector points. The figure also shows a cross section of a shielded polyurethane cable 23, with two wires for the (parallel coupled) Peltier elements 23 and two for the thermocouple 12 (for temperature feedback). Electrical current is supplied from the thermostimulation apparatus 1 to the Peltier elements 23 via connector 27

The thermostimulation apparatus described in this application may comprise a control system and/or one or more pads with temperature feedback as described in any one of US patent applications US 12/592,498, US 592,492, US 12/592,470 and US 12/592,493.

The thermostimulation apparatus and the pads described in this application are particularly advantageous for the treatment of muscular injuries, rehabilitation, but also relaxation and massaging.

## Claims

1. A thermostimulation apparatus comprising means for providing heating and cooling.

2. The apparatus according to claim 1 comprising at least one heating pad and at least one cooling pad.

3. The apparatus according to claim 2, wherein the at least one heating pad comprises means for providing electrical stimulation and means for providing heat.

4. The apparatus according to claim 3, wherein the pad comprises a top layer, a bottom layer for contacting the area to be treated, and a first and a second middle layer for supporting the stimulation means and the heating means.

5. The apparatus according to claim 2, wherein the cooling pad comprises means to receive a refrigerant in a liquid state or in a gaseous state.

6. The apparatus according to claim 5, further comprising means to vaporise a liquid refrigerant.

7. The apparatus according to claim 1 or 2, comprising at least one pad capable of providing both heating and cooling.

8. The apparatus according to claim 7, wherein the pad comprises one or more Peltier elements.

9. The apparatus according to claim 7, wherein the pad comprises a heat sink means.

10. The apparatus according to claim 7, wherein the pad comprises a heat exchanger means.

11. The apparatus according to any one of claims 2 to 10, wherein the pad comprises a thermocouple connected to a central processing unit to regulate the temperature of the pad.

12. A pad as defined in any one of claims 2 to 10 for use with a thermostimulation apparatus.

13. A thermostimulation apparatus as described herein with reference to the accompanying figures.

14. A pad as described herein with reference to the accompanying figures, for use with a thermostimulation apparatus.
